# EUROPEAN PATENT APPLICATION

(11) **EP 3 964 520 A1**
(43) Date of publication of application: **09.03.2022**
(21) Application number: 20798519.3
(22) Date of filing: 29.04.2020
(51) Int. Cl.: C07K 7/06, A61K 38/17, A61P 35/00

(54) **NOVEL OLIGOPEPTIDE, AND PHARMACEUTICAL COMPOSITION FOR PREVENTING OR TREATING CANCER, COMPRISING SAME AS ACTIVE INGREDIENT**

(30) Priority: 02.05.2019 US 201962842155 P
(71) Applicant: L-Base Co.,Ltd., Seoul 04778 (KR)
(72) Inventor: JEON, Do Yong, Seoul 04778 (KR); WOO, Sang Hyeok, Seoul 04778 (KR); MOON, Chang Hoon, Seoul 04778 (KR); JUNG, Ji Eun, Seoul 04778 (KR); SHIN, Hyun Hee, Seoul 04778 (KR); LEE, Hyerim, Seoul 04778 (KR); LEE, Jee Young, Seoul 04778 (KR)
(74) Representative: Dehns
(86) International application number: PCT/KR2020/005759
(87) International publication number: WO 2020/222559

(57) **Abstract**

The present invention relates to a novel oligopeptide, and a pharmaceutical composition for preventing or treating cancer, comprising same as an active ingredient. The oligopeptide of the present invention has an advantage in that the molecular weight thereof is less than that of an antibody, and thus the risk of an immune response is low, and it has better tissue permeability. The oligopeptide can exhibit the effect of inhibiting the proliferation of malignant tumors by selectively acting on cancer cells or cancer tissue.

## Description

### [Technical Field]

The present invention relates to a novel oligopeptide, a pharmaceutical composition for preventing or treating cancer, comprising the same as an active ingredient, and a preparation method thereof.

The present application claims priority to US Provisional Application No. 62/842,155 filed on May 2, 2019, and all the contents disclosed in the specification and drawings of that application are incorporated by reference in its entirety.

### [Background Art]

Currently, even though the effect of the cancer treatment is been continously improved as a result of the progress of diagnosis methods for cancer in early stages and the development of new anticancer therapies, the cancer is still considered as an critical disease due to its ranking in the first or second place among the causes of death in Korea. The cancer treatment issue is the most of the anticancer drugs currently used are based on the chemotherapy, which have different pharmacological activity depending on the type of cancer, and various side effects due to its toxicity.

Existing anticancer drugs permeate not only into the cancer but also normal tissues and damage the cellular activities and functions, which causes side effects such as bone marrow dysfunction, gastrointestinal disturbance, alopecia, and the cancer treatment vast complications have being observed such as multi-drug resistance associated with long term chemotherapy. Therefore, studies on the development of innovative anticancer drugs capable of solving these serious problems of existing anticancer drugs are being actively conducted.

Meanwhile, despite the specific tumor cell antigen targeting antibody has been developed, there are difficulties related to antibodies such as low efficiency of tissue permeability and concerns about immune responses. In contrast, unlike antibodies, peptides have an advantage of less concerns about immune responses and better tissue permeability due to their small molecular weight. Therefore, it is been expected that peptide based anticancer drugs that targeting tumor antigen could selectively affect the cancer without having any side effects related to damages to normal cells.

### [Disclosure]

### [Technical Problem]

The present invention has been contrived to solve the problems in the related art as described above, and confirmed that six types of oligopeptides with a common sequence can effectively treat cancer by suppressing the proliferation of cancer cells and inducing apoptosis, thereby completing the present invention.

Accordingly, an objective of the present invention is to provide an oligopeptide represented by the following General Formula:

[General Formula] A-Q-T-X-T-G-K-T

in General Formula,
A is alanine; Q is glutamine; T is threonine; G is glycine; and K is lysine; and
X is one or more selected from the group consisting of isoleucine, leucine, alanine, methionine, proline, and valine.

Another objective of the present invention is to provide a pharmaceutical composition for preventing or treating cancer, comprising the oligopeptide represented by General Formula as an active ingredient.

However, the technical problems which the present invention intends to solve are not limited to the technical problems which have been mentioned above, and other technical problems which have not been mentioned will be apparently understood by a person with ordinary skill in the art to which the present invention pertains from the following description.

### [Technical Solution]

In order to achieve the objectives of the present invention as described above, the present invention provides an oligopeptide represented by the following General Formula:

[General Formula] A-Q-T-X-T-G-K-T

in General Formula,
A is alanine; Q is glutamine; T is threonine; G is glycine; and K is lysine; and
X is one or more selected from the group consisting of isoleucine, leucine, alanine, methionine, proline, and valine.

Further, the present invention provides a pharmaceutical composition for preventing or treating cancer, comprising an oligopeptide represented by the following General Formula as an active ingredient:

[General Formula] A-Q-T-X-T-G-K-T

in General Formula,
A is alanine; Q is glutamine; T is threonine; G is glycine; and K is lysine; and
X is one or more selected from the group consisting of isoleucine, leucine, alanine, methionine, proline, and valine.

Further, the present invention provides a method for preventing or treating cancer, the method comprising administering a composition comprising the oligopeptide represented by General Formula as an active ingredient to an subject.

Further, the present invention provides a method for enhancing the activity of an anticancer drug, the method comprising administering a composition comprising the oligopeptide represented by General Formula as an active ingredient to an subject.

In addition, the present invention provides a use of the oligopeptide represented by General Formula for preventing, alleviating, or treating cancer.

Furthermore, the present invention provides a use of the oligopeptide represented by General Formula for producing a drug used for cancer.

In an exemplary embodiment of the present invention, the cancer may be a cancer selected from the group consisting of lung cancer, breast cancer, blood cancer, colorectal cancer, pancreatic cancer, and combinations thereof, but is not limited thereto.

In another exemplary embodiment of the present invention, the lung cancer may be non-small cell lung cancer, but is not limited thereto.

In still another exemplary embodiment of the present invention, the breast cancer may be triple negative breast cancer, but is not limited thereto.

In yet another exemplary embodiment, the blood cancer may be a blood cancer selected from the group consisting of leukemia, lymphoma, multiple myeloma, and combinations thereof, but is not limited thereto.

In yet another exemplary embodiment of the present invention, X may be one or more selected from the group consisting of alanine, methionine, proline, and valine, and the cancer may be lung cancer, but is not limited thereto.

In yet another exemplary embodiment of the present invention, X may be one or more selected from the group consisting of isoleucine, leucine, alanine, and valine, and the cancer may be breast cancer, but is not limited thereto.

In yet another exemplary embodiment of the present invention, X is one or more selected from the group consisting of isoleucine, leucine, alanine, methionine, proline, and valine, and the cancer may be a blood cancer, but is not limited thereto.

### [Advantageous Effects]

The present invention relates to a novel oligopeptide and a pharmaceutical composition for preventing or treating cancer, comprising the same as an active ingredient, and the pharmaceutical composition exhibits effects of suppressing the proliferation of cancer cells and inducing apoptosis, and thus can be used as a beneficial drug for treating cancer. The pharmaceutical composition of the present invention comprises one or more of six types of oligopeptides as an active ingredient, the oligopeptides have an advantage of less concerns about immune responses and better tissue permeability due to their small molecular weight compared to antibodies, thus expected to be able to effectively alleviate side effects of existing anticancer drugs due to its selective affect on the cancer cells and cancer tissues.

### [Description of Drawings]

FIG. 1 illustrates the results of MTT assay showing the proliferation suppressive effect of the oligopeptide according to the present invention on a lung cancer cell line A549 (^{∗}p<0.05).
FIG. 2 illustrates the results of MTT assay showing the proliferation suppressive effect of the oligopeptide according to the present invention on a lung cancer cell line H1650 (^{∗}p<0.05).
FIG. 3 illustrates the results of clonogenic assay showing the colony formation suppressive effect of the oligopeptide according to the present invention on a lung cancer cell line H1975 (^{∗∗}p<0.01).
FIG. 4 illustrates the results of clonogenic assay showing the colony formation suppressive effect of the oligopeptide according to the present invention on a breast cancer cell line MDA-MB-231.
FIG. 5 illustrates the results of CellTiter-Glo Luminescent (CTG) assay showing the cell activity suppressive effect of the oligopeptide according to the present invention on a breast cancer cell line HCC70 (^{∗}p<0.05).
FIG. 6 illustrates the results of MTT assay showing the proliferation suppressive effect of the oligopeptide according to the present invention on a blood cancer cell line JeKo-1 (^{∗}p <0.05, ^{∗∗}p <0.01).
FIG. 7 illustrates the results of MTT assay showing the proliferation suppressive effect of the oligopeptide according to the present invention on a blood cancer cell line Z-138 (^{∗}p <0.05, ^{∗∗}p <0.01).

### [Modes of the Invention]

The present inventors newly synthesized six types of oligopeptides with a common amino acid sequence of A-Q-T-X-T-G-K-T, and confirmed that the oligopeptides exhibit excellent anticancer effects on lung, breast, blood, colorectal and pancreatic cancers, thereby completing the present invention.

Thus, the present invention may provide an oligopeptide represented by the following General Formula:

[General Formula] A-Q-T-X-T-G-K-T

in General Formula,
A is alanine; Q is glutamine; T is threonine; G is glycine; and K is lysine; and
X is one or more selected from the group consisting of isoleucine, leucine, alanine, methionine, proline, and valine.

As another aspect of the present invention, the present invention may provide a pharmaceutical composition for preventing or treating cancer, comprising an oligopeptide represented by the following General Formula as an active ingredient:

[General Formula] A-Q-T-X-T-G-K-T

in General Formula,
A is alanine; Q is glutamine; T is threonine; G is glycine; and K is lysine; and
X is one or more selected from the group consisting of isoleucine, leucine, alanine, methionine, proline, and valine.

As still another aspect of the present invention, the present invention may provide a method for preventing or treating cancer, the method comprising administering a composition comprising the oligopeptide represented by General Formula as an active ingredient to an subject.

As yet another aspect of the present invention, the present invention may provide a use of the oligopeptide represented by General Formula for preventing, alleviating, or treating cancer.

As yet another aspect of the present invention, the present invention may provide a use of the oligopeptide represented by General Formula for producing a drug used for cancer.

As used herein, the term "prevention" refers to all actions that block, suppress or delay the symptoms caused by cancer by administering the composition according to the present invention.

As used herein, the term "treatment" refers to all actions that ameliorate or beneficially change symptoms caused by cancer by administering the composition according to the present invention.

As used herein, the term "subject" refers to a subject in need of prevention or treatment of a disease. For example, the subject may be a human or a mammal, including a non-human primate, a mouse, a dog, a cat, a horse, a sheep and a cow.

As used herein, the term "oligopeptide" refers to a linear molecule formed by bonding amino acid residues to each other by peptide bonds. The oligopeptide of the present invention may be prepared by a chemical synthesis method known in the art (for example, solid-phase synthesis techniques) together with a molecular biological method (Merrifield, J. Amer. Chem. Soc. 85: 2149-54(1963); Stewart, et al., Solid Phase Peptide Synthesis, 2nd. ed., Pierce Chem. Co.: Rockford, 111(1984)).

The scope of the compound according to the present invention may also include pharmaceutically acceptable salts thereof. As used herein, the term "pharmaceutically acceptable" refers to a compound which is suitable for use in contact with tissues of a subject (for example: a human) and within the scope of the sound medical judgment because its benefit/risk ratio is reasonable without excessive toxicity, irritation, allergic reactions or other problems or complications. The pharmaceutically acceptable salt includes, for example, acid addition salts formed by pharmaceutically acceptable free acids and pharmaceutically acceptable metal salts.

Further, the scope of the compound according to the present invention may include biologically functional equivalents with variations in the amino acid sequence that exert a biological activity equivalent to the compound of the present invention. Such variations in the amino acid sequence may be based on the relative similarity of side chain substituents of amino acids, such as hydrophobicity, hydrophilicity, charge and size. By the analysis of the size, shape and type of side chain substituents of amino acids, it can be seen that alanine and glycine have similar sizes; lysine is a positively charged residue; and glutamine and threonine are not charged. Accordingly, based on these considerations, alanine and glycine; and glutamine and threonine are biologically functional equivalents.

In introducing the variation, the hydropathic index of the amino acid can be considered. Each amino acid is assigned a hydropathic index as follows, depending on its hydrophobicity and charge: isoleucine (+4.5); valine (+4.2); leucine (+3.8); phenylalanine (+2.8); cysteine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamic acid (-3.5); glutamine (-3.5); aspartic acid (-3.5); asparagine (-3.5); lysine (-3.9); and arginine (-4.5).

The hydropathic index of an amino acid is very important in imparting the interactive biological function of proteins. It is known that substitution with an amino acid having a similar hydropathic index can retain similar biological activity. When variations are introduced with reference to the hydropathic index, substitutions are made between amino acids showing a hydropathic index difference preferably within ±2, more preferably within ±1, and even more preferably within ±0.5.

Meanwhile, it is also well known that substitutions between amino acids with similar hydrophilicity values result in proteins with equivalent biological activity. As disclosed in U.S. Patent No. 4,554,101, the following hydrophilicity values are assigned to each amino acid residue: arginine (+3.0); lysine (+3.0); aspartic acid (+3.0±1); glutamic acid (+3.0±1); serine (+0.3); asparagine (+0.2); glutamine (+0.2); glycine 0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4).

When variations are introduced with reference to the hydrophilicity value, substitutions are made between amino acids showing a hydrophilicity value difference preferably within ±2, more preferably within ±1, and even more preferably within ±0.5.

Amino acid exchanges in proteins that do not totally alter the activity of the molecule are known in the art (H. Neurath, R.L.Hill, The Proteins, Academic Press, New York, 1979). The most typically occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

In consideration of the variations having the above-described biologically equivalent activity, the oligopeptide of the amino acid sequence represented by General Formula (AQTXTGKT) of the present invention is also interpreted to include a sequence showing substantial identity. The aforementioned substantial identity refers to a sequence showing at least 62.5% homology, more preferably 75% or more homology, and most preferably 87.5% or more homology to the sequence of the present invention, when it is aligned to correspond to the sequence of the present invention as much as possible, and the aligned sequences are analyzed using an algorithm typically used in the art. Alignment methods for sequence comparison are known in the art.

The pharmaceutical composition of the present invention is used for the prevention or treatment of cancer. Cancers for which the pharmaceutical composition of the present invention can be used may be a cancer selected from the group consisting of lung cancer, breast cancer, blood cancer, colorectal cancer, pancreatic cancer, and combinations thereof, but is not limited thereto.

In the present invention, the lung cancer may be non-small cell lung cancer, but is not limited thereto.

Further, the breast cancer may be triple negative breast cancer, but is not limited thereto.

In addition, although the blood cancer may be a blood cancer selected from the group consisting of leukemia, lymphoma, multiple myeloma, and combinations thereof, the lymphoma may be Non-Hodgkin's lymphoma, but is not limited thereto.

In an exemplary embodiment of the present invention, it was confirmed that the pharmaceutical composition according to the present invention exhibits excellent anticancer activity against lung cancer, breast cancer and blood cancer (see Example 2).

In the present invention, when the pharmaceutical composition is used for the prevention or treatment of lung cancer, the pharmaceutical composition includes an oligopeptide represented by General Formula AQTXTGKT as an active ingredient, and in this case, X may be one or more selected from the group consisting of isoleucine, leucine, alanine, methionine, proline and valine, but is not limited thereto.

In addition, in the present invention, when the pharmaceutical composition is used for the prevention or treatment of lung cancer, the pharmaceutical composition includes an oligopeptide represented by General Formula AQTXTGKT as an active ingredient, and in this case, wherein the X may be one or more selected from the group consisting of alanine, methionine, proline and valine, but is not limited thereto.

Furthermore, in the present invention, when the pharmaceutical composition is used for the prevention or treatment of breast cancer, the pharmaceutical composition includes an oligopeptide represented by General Formula AQTXTGKT as an active ingredient, and in this case, wherein the X may be one or more selected from the group consisting of isoleucine, leucine, alanine, methionine, proline and valine, but is not limited thereto.

Further, in the present invention, when the pharmaceutical composition is used for the prevention or treatment of breast cancer, the pharmaceutical composition includes an oligopeptide represented by General Formula AQTXTGKT as an active ingredient, and in this case, wherein the X may be one or more selected from the group consisting of isoleucine, leucine, alanine, and valine, but is not limited thereto.

In addition, in the present invention, when the pharmaceutical composition is used for the prevention or treatment of blood cancer, the pharmaceutical composition includes an oligopeptide represented by General Formula AQTXTGKT as an active ingredient, and in this case, wherein the X may be one or more selected from the group consisting of isoleucine, leucine, alanine, methionine, proline and valine, but is not limited thereto.

As still another aspect of the present invention, the present invention may provide a method for enhancing the activity of an anticancer drug, the method comprising administering a composition comprising the oligopeptide represented by General Formula as an active ingredient to an subject.

In the present invention, the composition can enhance the anticancer activity of an anticancer drug by reducing the resistance or tolerance of cancer tissue or cancer cells to the anticancer drug.

Meanwhile, the pharmaceutical composition according to the present invention may further include a suitable carrier, excipient and/or diluent which are/is typically used for preparation as a pharmaceutical composition in addition to the active ingredient. Further, the pharmaceutical composition may be used by being formulated in the form of an oral formulation such as a powder, granules, a tablet, a capsule, a suspension, an emulsion, a syrup, and an aerosol, an external preparation, a suppository, and a sterile injection solution, according to a typical method.

Examples of the carrier, the excipient, and the diluent, which may be included in the composition include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, and the like. When the composition is prepared, the composition may be prepared using a commonly used diluent or excipient, such as a filler, an extender, a binder, a wetting agent, a disintegrant, and a surfactant.

The pharmaceutical composition according to the present invention is administered in a pharmaceutically effective amount. In the present invention, the pharmaceutically effective amount refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and other factors well known in the medical field. A preferred dosage of the preparation of the present invention may be selected depending on the condition and body weight of an subject, the degree of a disease, the form of drug, the administration route, and the duration. As a specific example, the pharmaceutical composition may be administered in an amount of 0.001 to 1000 mg/kg, 0.01 to 100 mg/kg, 0.01 to 10 mg/kg, 0.1 to 10 mg/kg or 0.1 to 1 mg/kg once or several times per day.

It is important to administer the composition in a minimum amount that can obtain the maximum effect without any side effects, in consideration of all the aforementioned factors, and this amount may be determined by those skilled in the art. Specifically, the effective amount of the pharmaceutical composition according to the present invention may vary depending on the age, sex, condition, and body weight of a patient, the absorption rate, inactivation rate and excretion rate of the active ingredient *in vivo*, the type of the disease, and the drug to be used in combination.

The pharmaceutical composition of the present invention may be administered to an subject via various routes. For example, the pharmaceutical composition may be administered, for example, by oral administration, intranasal administration, transtracheal administration, arterial injection, intravenous injection, subcutaneous injection, intramuscular injection, or intraperitoneal injection. The daily dosage may be administered once or in several divided doses per day.

Terms or words used in the specification and the claims should not be interpreted as being limited to a typical or dictionary meaning and should be interpreted with a meaning and a concept which conform to the technical spirit of the present invention based on the principle that an inventor can appropriately define a concept of a term in order to describe his/her own invention by the best method.

Hereinafter, preferred examples for helping the understanding of the present invention will be suggested. However, the following examples are provided only to more easily understand the present invention, and the contents of the present invention are not limited by the following examples.

### [Examples]

### Experimental method

### 1. CellTiter-Glo Luminescent (CTG) assay

Cancer cell lines were treated with six types of oligopeptides AQTITGKT, AQTLTGKT, AQTATGKT, AQTMTGKT, AQTPTGKT and AQTVTGKT according to the present invention, and the level of cell proliferation was measured by CTG assay.

Specifically, cells were seeded on a 96-well plate at 5 × 10³ cells/100 µl per well, cultured for 24 hours, and then transfected with six types of oligopeptides according to the present invention. Cells without the oligopeptides transfection and cultured at the same condition were used as control. After 72 hours, a CellTiter-Glo (Promega Co., USA) reagent was mixed in the same amount as the cell culture medium and allowed to react in an orbital shaker for 2 minutes. After incubating at room temperature for 10 minutes, the luminescence signal was measured by using a luminometer (GloMax Promega).

### 2. Clonogenic assay

For clonogenic assay, cells were detached from culture dish by using trypsin/EDTA when confluence reached 80% to 90%. The detached cells were resuspendend in a 50 ml tube, and then cells were counted after diluted with 2-folds of trypan blue. The 1.7 × 10⁵ cells/2 ml per well were seeded on a 6-well plate (SPL #30006, flat bottom) for 24 hours, and then transfected cells with six types of oligopeptides according to the present invention. Cells without the oligopeptides transfection and cultured at the same condition were used as control.

More specifically, the oligopeptides were added to 200 µl of j etPrime buffer to make a final concentration of 100 µM, mixed well, spun down, and incubated at room temperature for 10 minutes. 200 µl of buffer mixture were added to each well and cultured at 5% CO₂ and 37°C for 24 hours. And then, 1 × 10³ cells/ml were added to each well and transfected for 14 days. After transfection, the cells were stained and observed.

### 3. Tetrazolium (MTT) assay

Cancer cell lines were treated with six types of oligopeptides according to the present invention and the level of cell proliferation was measured by MTT assay. Specifically, 5 × 10³cells/100 µl per well seeded into a 96-well plate for 24 hours, and then treated with the oligopeptides AQTITGKT, AQTLTGKT, AQTATGKT, AQTMTGKT, AQTPTGKT and AQTVTGKT according to the present invention for 72 hours. Cells without the oligopeptides transfection and cultured at the same condition were used as control. After treatment, 10 µl of a CellTiter-96 (Promega Co., USA) reagent was added to each well and incubated at 5% CO₂ and 37°C for 3 hours. After incubation, absorbance was measured at 490 nm using a spectrophotometer (SPECTROstar^{Nano}, BMG).

### Example 1: Preparation of oligopeptides

### 1.1. Reaction in general

All reactions were performed using commercially available materials and reagents without additional reactions, unless otherwise stated. All oligopeptide products were specified using liquid chromatography tandem-mass spectrometry (LC-MS/MS), high performance liquid chromatography (HPLC), and/or sequencing.

Specifically, LC-MS/MS and De novo sequencing were performed using LTQ XL MS/MSn spectrometer (Thermo Fisher Scientific, USA) and Discoverer 2.3 (Thermo Fisher Scientific). HPLC was performed using Agilent 1100 HPLC System (Agilent Technologies, USA) (detector, VWD detector, 220 nm; column, Waters XBridge^{®} C18, 4.6 mm i.d. × 250 mm, 5 µm; A, 0.1% TFA in Water; B, 0.1% TFA in ACN, v/v; flow rate, 1 mL/min; Gradient, B: 5% - 35%, within 5 minutes, B: 35% - 65%, within 15 minutes). All analyses were performed by commissioning SEWON BIOTECH.

### 1.2. Synthesis and confirmation of 6 types of oligopeptides

The amino acid sequence and structure of synthesized oligopeptides are shown in the Table 1. Oligopeptides were synthesized by commissioning SEWON BIOTECH, amino acid sequence were identified by the LC-MS/MS, and confirmed by the HPLC with above 95% of purity (see the Table 2).

**[Table 1]**

| **SEQ ID NO** | **Amino acid Sequence** | **Chemical structure** |
|---|---|---|
| SEQ ID NO: 1 (Oligopeptide #1) | AQTITGKT | 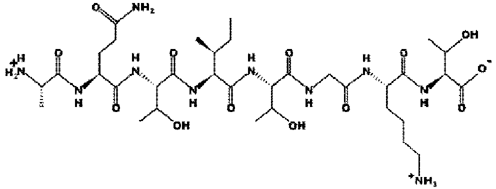 |
| SEQ ID NO: 2 (Oligopeptide #2) | AQTLTGKT | 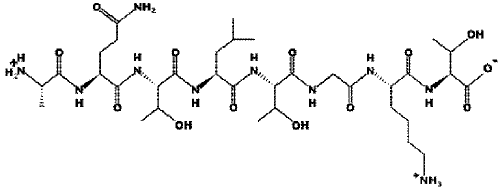 |
| SEQ ID NO: 3 (Oligopeptide #3) | AQTATGKT | 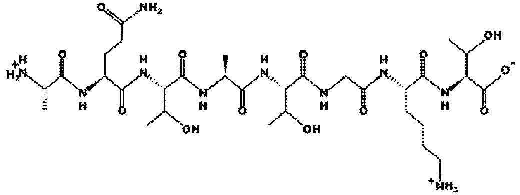 |
| SEQ ID NO: 4 (Oligopeptide #4) | AQTMTGKT | 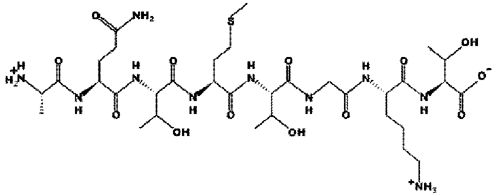 |
| SEQ ID NO: 5 (Oligopeptide #5) | AQTPTGKT | 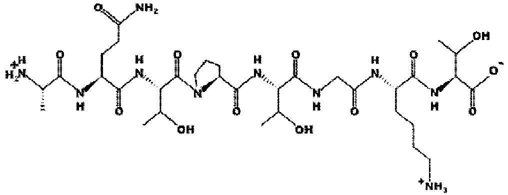 |
| SEQ ID NO: 6 (Oligopeptide #6) | AQTVTGKT | 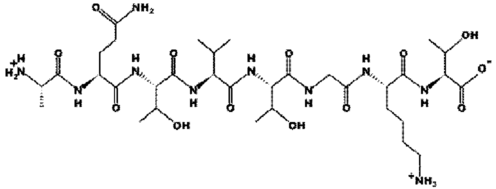 |

**[Table 2]**

| Oligopeptide | Purity (purity; by analytic HPLC) | Identity (identity; by LC-MS/MS and Sequence Analysis) |
|---|---|---|
| AQTITGKT (SEQ ID NO: 1) | 97.6% | 100% |
| AQTLTGKT (SEQ ID NO: 2) | 97.36% | 100% |
| AQTATGKT (SEQ ID NO: 3) | 97.8% | 100% |
| AQTMTGKT (SEQ ID NO: 4) | 96.5% | 100% |
| AQTPTGKT (SEQ ID NO: 5) | 97.6% | 100% |
| AQTVTGKT (SEQ ID NO: 6) | 97.6% | 100 % |

Specifically, the oligopeptides of SEQ ID NOS: 1 to 6 were synthesized by a solid phase synthesis method using fluorenylmethyloxycarbonyl (Fmoc)/t-Bu. The oligopeptides were synthesized using a 24-row peptide automatic synthesizer Syro-I (Biotage, Sweden), and 2-chlorotrityl resin was used. The first amino acid at the C-terminus was introduced separately and attached to the synthesizer.

The Fmoc-amino acid was introduced using diisopropylcarbodiimide/1-hydroxybenzotriazole/diisopropylethylamine (DIC/HOBT/DIPEA) conditions, and the protecting group Fmoc of the N-amino group was removed using a 20% piperidine-DMF solution.

The peptides were isolated from the resin by treatment with a trifluoroacetic acid (TFA)-based solution [TFA: triisopropylsilane (TIS) : water = 95 : 2.5 : 2.5], and then a peptide mixture was obtained as a precipitate using an excessive amount of diethyl ether.

The peptide mixture was analyzed using HPLC (Agilent1100, USA) and LC/MS (Thermo Scientific LTQ XL^{™} IonTrap Mass Spectrometer, USA) and purified using prep-HPLC (Waters 2998, USA), and a purified peptide solution was concentrated using a lyophilizer. The peptide was substituted with acetate by dissolving the lyophilized peptide in an acetic acid solution and allowing the dissolved solution to pass through an anion exchange resin (Dowex 1X8 chloride form, 100 to 200 mesh), and the resulting product was finally dried to obtain a peptide powder.

The purity of the peptide was analyzed by HPLC, mass spectrometry was performed by LCMS, and sequence analysis was performed by a De novo sequence analysis method using the Thermo Xcalibur Qual Browser program.

### Example 2: Confirmation of anticancer effects of oligopeptides

### 2.1. Effects on suppression of proliferation of lung cancer cells and suppression of formation of colonies

As described in the experimental methods by the MTT and clonogenic assays, non-small cell lung cancer cell lines A549, H1650 and H1975 were treated with the oligopeptide AQTITGKT, AQTLTGKT, AQTATGKT, AQTMTGKT, AQTPTGKT or AQTVTGKT according to the present invention, at the concentration of 100 µM or 500 µM, and the level of colony formation and cytotoxicity were measured.

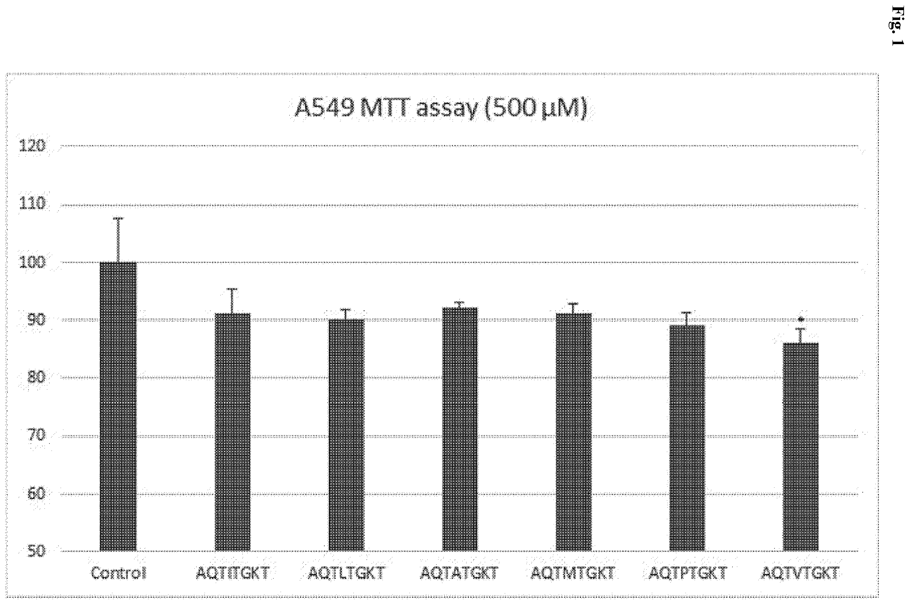

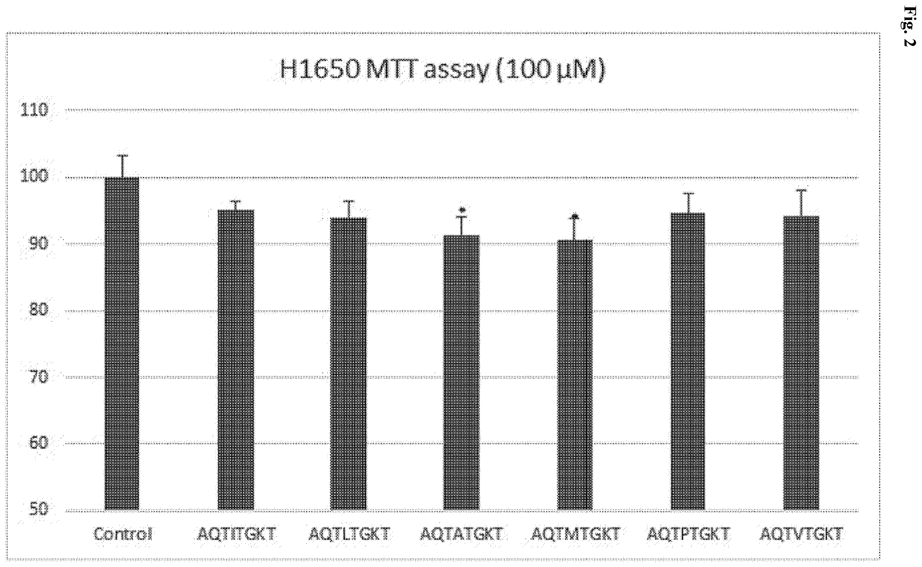

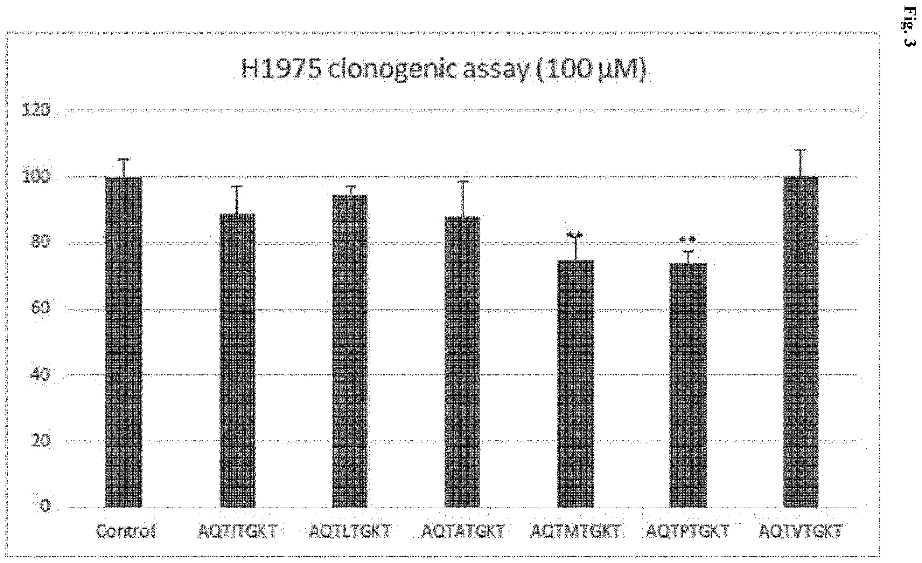

As result illustrated in FIGS. 1 to 3, the cell lines treated with AQTATGKT, AQTMTGKT, AQTPTGKT or AQTVTGKT had shown significant cytotoxicity and colony formation suppressive effects compared to those of the control group. These results suggest that the oligopeptides specified in the present invention could have an anticancer effect on lung cancer cells by suppressing the cell growth and proliferation and colony formation.

### 2.2. Effects on suppression of proliferation and cellular activity of breast cancer cells

As described in the experimental methods by the CTG and clonogenic assays, breast cancer cell lines MDA-MB-231 and HCC70 were treated with the oligopeptide AQTITGKT, AQTITGKT, AQTLTGKT, AQTATGKT, AQTMTGKT, AQTPTGKT or AQTVTGKT according to the present invention, at a concentration of 100 µM or 500 µM, and the anticancer effects were analyzed by measuring the level of colony formation and measuring the amount of ATP in the cells.

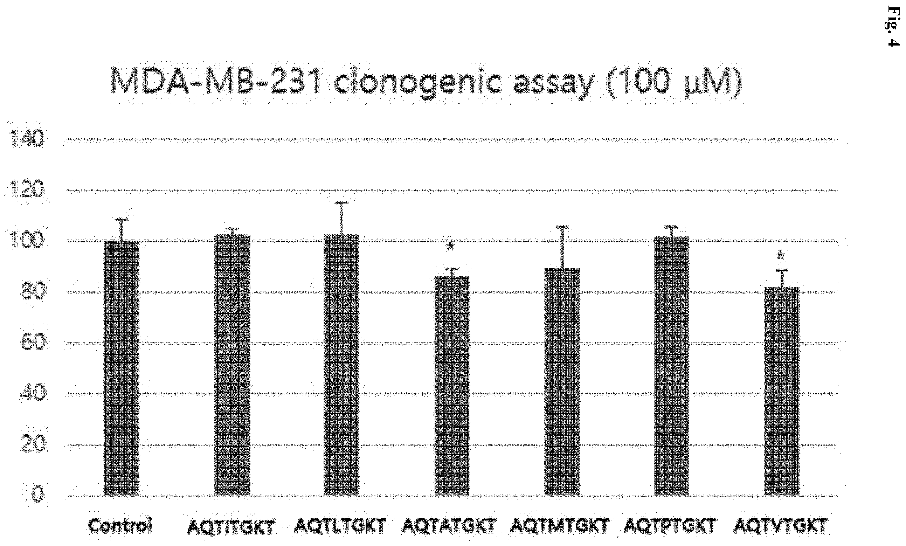

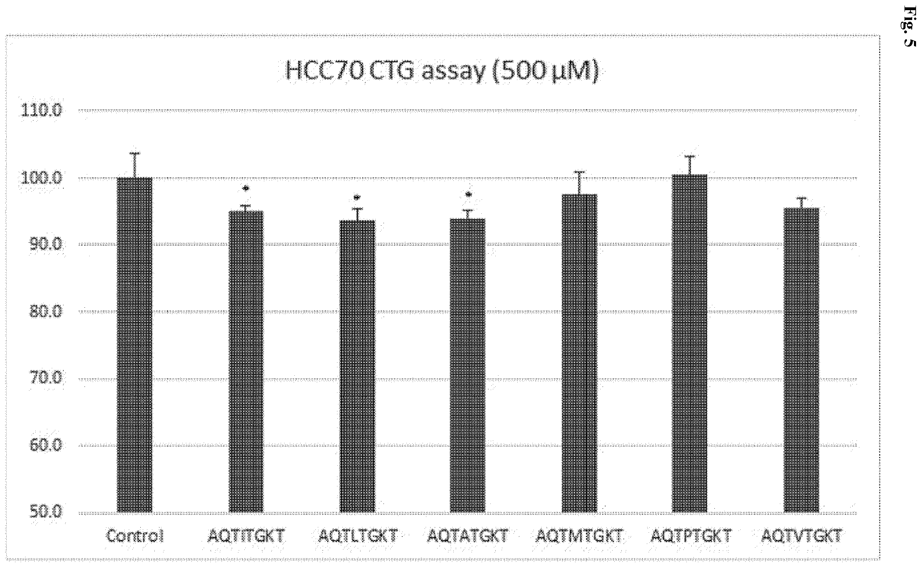

As result illustrated in FIGS. 4 to 5, cell lines were treated with AQTITGKT, AQTLTGKT, AQTATGKT or AQTVTGKT had shown significant effects on suppression of the colony formation and the activity of cancer cells compared to those of the control group. These results suggest that the oligopeptide specified in the present invention could have an anticancer effect on breast cancer cells by suppressing the cell proliferation and cellular activity.

### 2.3. Effects on suppression of growth and proliferation ability of blood cancer cells

As described in the experimental methods by the MTT assay, blood cancer cell lines Z-138 and JeKo-1 were treated with the oligopeptide AQTITGKT, AQTLTGKT, AQTATGKT, AQTMTGKT, AQTPTGKT or AQTVTGKT according to the present invention, at a concentration of 100 µM or 500 µM, and cytotoxicity were measured.

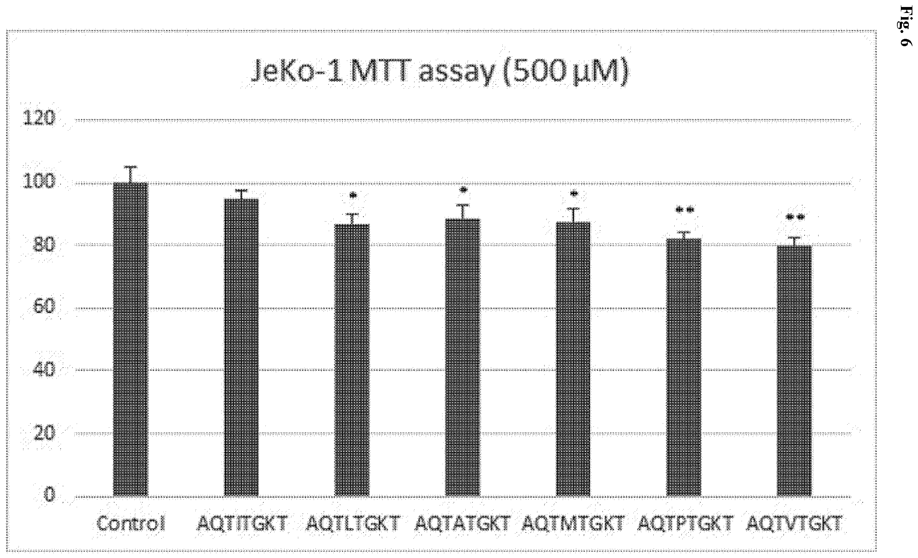

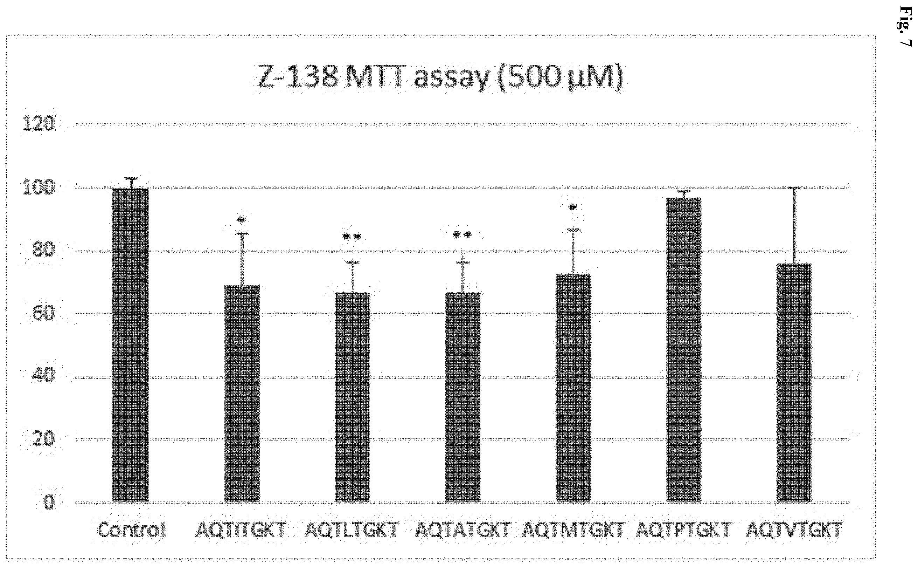

As result illustrated in FIGS. 6 and 7, all 6 types of oligopeptides showed cytotoxicity effects, which indicate that the oligopeptide specified in the present invention has an excellent anticancer effect particularly on blood cancer cells.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only exemplary in all aspects and are not restrictive.

### [Industrial Applicability]

The oligopeptide according to the present invention and the pharmaceutical composition including the same as an active ingredient exhibit effects of suppressing the proliferation of cancer cells and inducing apoptosis, and thus can be used as a useful anticancer drug for treating cancer. The pharmaceutical composition of the present invention includes the oligopeptide as an active ingredient, and the oligopeptides have an advantage of less concerns about immune responses and easy penetration into tissues due to their small molecular weight compared to antibodies, and can selectively act on cancer cells or cancer tissues, and thus are expected to be able to effectively alleviate side effect problems of existing anticancer drugs.

## Claims

1. An oligopeptide represented by the following General Formula:
[General Formula] A-Q-T-X-T-G-K-T
in General Formula,
A is alanine; Q is glutamine; T is threonine; G is glycine; and K is lysine; and
X is one or more selected from the group consisting of isoleucine, leucine, alanine, methionine, proline, and valine.

2. A pharmaceutical composition for preventing or treating cancer, comprising an oligopeptide represented by the following General Formula as an active ingredient:
[General Formula] A-Q-T-X-T-G-K-T
in General Formula,
A is alanine; Q is glutamine; T is threonine; G is glycine; and K is lysine; and
X is one or more selected from the group consisting of isoleucine, leucine, alanine, methionine, proline, and valine.

3. The pharmaceutical composition of claim 2, wherein the cancer is a cancer selected from the group consisting of lung cancer, breast cancer, blood cancer, colorectal cancer, pancreatic cancer, and combinations thereof.

4. The pharmaceutical composition of claim 3, wherein the lung cancer is non-small cell lung cancer.

5. The pharmaceutical composition of claim 3, wherein the breast cancer is triple negative breast cancer.

6. The pharmaceutical composition of claim 3, wherein the blood cancer is a blood cancer selected from the group consisting of leukemia, lymphoma, multiple myeloma, and combinations thereof.

7. The pharmaceutical composition of claim 2, wherein the X is one or more selected from the group consisting of alanine, methionine, proline, and valine, and the cancer is lung cancer.

8. The pharmaceutical composition of claim 2, wherein the X is one or more selected from the group consisting of isoleucine, leucine, alanine, and valine, and the cancer is breast cancer.

9. The pharmaceutical composition of claim 2, wherein the X is one or more selected from the group consisting of isoleucine, leucine, alanine, methionine, proline, and valine, and the cancer is blood cancer.

10. A method for preventing or treating cancer, comprising administering a composition comprising an oligopeptide represented by the following General Formula as an active ingredient to an subject:
[General Formula] A-Q-T-X-T-G-K-T
in General Formula,
A is alanine; Q is glutamine; T is threonine; G is glycine; and K is lysine; and
X is one or more selected from the group consisting of isoleucine, leucine, alanine, methionine, proline, and valine.

11. A method for enhancing the activity of an anticancer drug, comprising administering a composition comprising an oligopeptide represented by the following General Formula as an active ingredient to an subject:
[General Formula] A-Q-T-X-T-G-K-T
in General Formula,
A is alanine; Q is glutamine; T is threonine; G is glycine; and K is lysine; and
X is one or more selected from the group consisting of isoleucine, leucine, alanine, methionine, proline, and valine.

12. A use of an oligopeptide represented by the following General Formula for preventing, alleviating or treating cancer:
[General Formula] A-Q-T-X-T-G-K-T
in General Formula,
A is alanine; Q is glutamine; T is threonine; G is glycine; and K is lysine; and
X is one or more selected from the group consisting of isoleucine, leucine, alanine, methionine, proline, and valine.

13. A use of an oligopeptide represented by the following General Formula for producing a drug used for cancer:
[General Formula] A-Q-T-X-T-G-K-T
in General Formula,
A is alanine; Q is glutamine; T is threonine; G is glycine; and K is lysine; and
X is one or more selected from the group consisting of isoleucine, leucine, alanine, methionine, proline, and valine.


<110>    L-Base Co., Ltd.

<120>    Novel oligopeptides and pharmaceutical composition for preventing
         or treating cancer comprising the same as an active ingredient

<130>    MPCT20-037

<150>    US 62/842,155
<151>    2019-05-02

<160>    6

<170>    KoPatentIn 3.0

<210>    1
<211>    8
<212>    PRT
<213>    Artificial Sequence

<220>
<223>    Oligopeptide #1 AQTITGKT


<400>    1
Ala Gln Thr Ile Thr Gly Lys Thr
  1               5


<210>    2
<211>    8
<212>    PRT
<213>    Artificial Sequence

<220>
<223>    Oligopeptide #2 AQTLTGKT


<400>    2
Ala Gln Thr Leu Thr Gly Lys Thr
  1               5


<210>    3
<211>    8
<212>    PRT
<213>    Artificial Sequence

<220>
<223>    Oligopeptide #3 AQTATGKT


<400>    3
Ala Gln Thr Ala Thr Gly Lys Thr
  1               5


<210>    4
<211>    8
<212>    PRT
<213>    Artificial Sequence

<220>
<223>    Oligopeptide #4 AQTMTGKT

<400>    4
Ala Gln Thr Met Thr Gly Lys Thr
1               5

<210>    5
<211>    8
<212>    PRT
<213>    Artificial Sequence

<220>
<223>    Oligopeptide #5 AQTPTGKT

<400>    5
Ala Gln Thr Pro Thr Gly Lys Thr
1               5

<210>    6
<211>    8
<212>    PRT
<213>    Artificial Sequence

<220>
<223>    Oligopeptide #6 AQTVTGKT

<400>    6
Ala Gln Thr Val Thr Gly Lys Thr
1               5